# EUROPEAN PATENT APPLICATION

(11) **EP 3 410 397 A1**
(43) Date of publication of application: **05.12.2018**
(21) Application number: 17743770.4
(22) Date of filing: 09.01.2017
(51) Int. Cl.: G06T 7/60, A61B 3/10, A61B 3/18, G02C 5/00, G02C 7/00

(54) **METHOD FOR PRODUCING LENSES AND FRAMES WITH PERSONALISED DESIGN AND REMOTE BEVELLING OF CORRECTIVE LENSES, AND OPTICAL DEVICE FOR MORPHOLOGICAL MEASUREMENT FOR PERFORMING SAID METHOD**

(30) Priority: 25.01.2016 ES 201630086; 28.12.2016 ES 201631703
(71) Applicant: Ópticas Claravisión, S.L., 18005 Granada (ES)
(72) Inventor: PÉREZ ORTEGA, Antonio Javier, 18005 Granada (ES); CANTÓ VAÑÓ, Javier, 18005 Granada (ES); GONZÁLEZ MERINO, Ramón, 18005 Granada (ES)
(74) Representative: Pons Ariño, Angel
(86) International application number: PCT/ES2017/070009
(87) International publication number: WO 2017/129842

(57) **Abstract**

The invention relates to a method for producing lenses and frames, with personalised design and remote bevelling of corrective lenses, and to an optical device for morphological measurement for performing said method. The method comprises the steps of: obtaining 3D morphological data; generating a 3D virtual model of a frame in a photographic image; capturing data, viewing and modifying frames; adjusting size and design; generating a data file for the frame in a compatible format for production by means of a 3D printer; obtaining a 3D image of an existing frame; capturing measurement data of the frame; and generating a file compatible with an edger. The device comprises: a 3D scanner (2); a photographic camera (3); a CPU (4); a tactile screen (11) and/or keyboard on a fixed structure (5) such as a column or a fixed structure secured to a wall (52) and having an L-shaped arm (91) with a rotary part (92) that rotates 360° and a longitudinal guide (93) for vertical movement.

## Description

### OBJECT OF THE INVENTION

The invention, as expressed in the title of the present specification, relates to a method for producing lenses and frames with personalised design and remote bevelling of corrective lenses, and to an optical device for morphological measurement for performing said method contributing, to the function for which it is intended, certain advantages and characteristics which will be described in detail below and represent a remarkable innovation in the current state of the art.

More specifically, the object of the invention focuses on a method and an application device within the optical industry which essentially comprises a 3D scanner, a photographic camera and various specific software programmes, and which is designed to obtain the facial morphology of a patient/customer in 3D with millimetric measurements for the purposes of producing personalised corrective lenses and for creating the frames for these lenses of the size and shape selected by the patient after designing the same on a personalised basis and trying them on virtually on the 3D image obtained by automatically generating the corresponding file, which is compatible with 3D printers.

In particular, the configuration of said device and the movement capacity of the mobile structure comprising and which incorporates the 3D scanner and the photographic camera are designed such that they can rotate 360° and, at the same time, move vertically around the patient's bust, making it possible to take measurements for the purpose of forming a perfect mesh of the patient's ear, nose, neck and head as necessary to obtain the measurement of the pantoscopic angle and the optometric measurements required for producing and bevelling lenses and for the virtual test of the eyeglasses to match the actual eyeglasses.

Thus, the method and the device also make it possible to obtain not only the eyeglasses personalised by the system but any frame provided by the customer in the exact same shape and, by means of a specific software, a file compatible with the edgers available in the market, which will contain all the parameters needed to cut the glass to that it fits the shape of the frame in a process known as bevelling. This bevelling process can be done in-store or remotely.

### FIELD OF APPLICATION OF THE INVENTION

The field of application of the present invention is the optical industry, and it focuses on the part of the industry engaged in the production of equipment, apparatuses and devices for morphological measurement intended for the production of lenses, as well as those intended for the production of frames.

### BACKGROUND OF THE INVENTION

It is a well-known fact that the production and bevelling of personalised corrective lenses above all, and of all corrective lenses in general, requires taking measurements of the facial morphology of the user, which are unique to each person and eyeglasses chosen. In particular, the measurements taken are: interpupillary distance; pantoscopic angle; Galbe angle; vertex distance; bend angle; reading distance, frame shape and pupil position.

At present, there are several devices available for taking measurements to produce prescription lenses.

These devices operate by taking photographs of the patient wearing eyeglasses that have certain predetermined markers (which are usually five). The 2D images obtained are combined with the markers on the eyeglasses, which serve as a reference for estimating the necessary measurements to produce the respective lenses.

The problem is that these devices perform an estimation of those values, which gives rise to numerous errors since they depend, to a significant extent, on the position of the patient/customer's head at that moment. In fact, the manufacturers of such devices point out that adjustment errors can cause a loss of up to 40% in lens performance.

As an example of these common errors, we have attached three tests conducted on the same day and on the same subject. The tests are identified with codes 0052, 0053 and 0055 and, despite being performed on the same individual, yield very different results:

| | **PANTOSCOPIC ANGLE** | **GALBE ANGLE** | **INTERPUPILLARY DISTANCE** RIGHT/LEFT EYE | **HEIGHT** RIGHT/LEFT EYE | **VERTEX DISTANCE** RIGHT/LEFT EYE |
|---|---|---|---|---|---|
| 0052 | 13.5º | 5.9º | 32.6/30.80 | 22.5/25.4 | 16.7/16.5 |
| 0053 | 10º | 5.8º | 32.7/30.8 | 19.8/21.8 | 16.7/16.8 |
| 0055 | -1.2º | 2.4º | 33.1/30.5 | 14.4/16.6 | 14.4/13.5 |

It is clear that the results are very different depending on the inclination or position of the patient/customer's eyes when taking the corresponding photographs.

In these tests, the variations in the patient/customer's position are not very marked, but there are cases in which the patient/customer's posture during the data capture process, either due to the position that the patient/customer has to adopt (the patient/customer must be standing and follow the marks on the floor and on the screen) or due to the stress of being tested, is very different from the patient/customer's usual position in his/her daily activities.

This means that the measurements taken are not very consistent with reality, due to which any lenses manufactured based on these measurements will not be a good fit for the patient/customer and it will be difficult for him/her to adapt to them. This adaptation problem can be much more severe in the case of progressive lenses.

The object of the present invention is, therefore, to develop an improved measurement device for obtaining morphological measurements that makes it possible to adjust such measurements to reality in a much more accurate manner and consequently produce lenses that meet the specific needs of each patient much more precisely.

On the other hand, and as a reference to the current state of the art, it should be noted that even through there are devices for ophthalmological measurement that are known and available in the market, at least as far as the applicant knows, there are none which exhibit technical, structural and constituent characteristics similar to those offered by the invention highlighted herein, as claimed.

### EXPLANATION OF THE INVENTION

Thus, the method for producing lenses and frames with personalised design and remote bevelling of corrective lenses, and the optical device for morphological measurement for performing said method proposed by the invention represent an innovation within their field of application since, according to a practical embodiment of the invention and in an exhaustive manner, the above-mentioned objectives are satisfactorily achieved with the characterising details that distinguish and make the invention possible, which are conveniently included in the final claims provided in the present description.

Specifically, and as noted above, the invention proposes a device which essentially comprises a 3D scanner, a photographic camera and specific software programmes and makes it possible to obtain the facial morphology of a patient/customer in 3D with millimetric measurements that are used both for the production and for the bevelling and subsequent assembly of corrective lenses of any kind.

Furthermore, such device can also be used for producing and creating personalised frames with 3D printers, as it makes it possible to create a frame of the size and shape selected by the customer.

To achieve this, after obtaining the morphological data of the face through a scan of the customer's bust and adding the corresponding texture with a photograph, it is possible to try on eyeglass models on a 3D mesh that the customer can digitally modify on his/her scanned face. At the end, the customer will be able to choose a certain frame size and shape, which have been virtually tested on his/her scanned image, and through the specific software, generate a data file compatible with a 3D printer to print the same on said type of devices and obtain a fully personalised frame.

To achieve this, after obtaining the morphological data of the face through a scan of the customer's bust and adding the corresponding texture with a photograph, it is possible to try on eyeglass models on a 3D mesh that the customer can digitally modify on his/her scanned face. At the end, the customer will be able to choose a certain frame size and shape, which have been virtually tested on his/her scanned image, and through the specific software, generate a data file compatible with a 3D printer to print the same on said type of devices and obtain a fully personalised frame.

Likewise, after obtaining the morphological data of the customer's face, he/she will be able to obtain all the necessary measurements for producing personalised glasses and bevelling all corrective lenses.

In addition, the device can also take the measurements of eyeglasses provided by the customer and obtain all the necessary parameters for the remote bevelling thereof.

To achieve this and more specifically, the device of the invention is composed of the following main elements:
- A fixed structure to which the rest of the elements of the device are attached, at least provided with one raised support for placing the 3D scanner and the photographic camera at the height of the customer's head. Said structure secures the scanning system, providing it with stability. Preferably, it constitutes a structural assembly mounted on the floor or from the ceiling.
- A mobile structure to which the 3D scanner is attached and which is provided with automatic or manual actuating means to move said scanner vertically and place it at the appropriate height although, optionally, it may also move horizontally to rotate around the customer's head or bust. Preferably, this mobile structure is a circular body that moves up and down and rotates on itself around the patient/customer's head, and which inner face is adapted to ensure outdoor light and possible reflections do not affect the capture of data by the scanner, as well as to ensure the lighting is appropriate.
- A 3D scanner, incorporated, as has been pointed out above, into the mobile structure, which constitutes the electronic device that will take the actual measurements of the patient's head or bust, including those of the face, for generating a 3D image thereof with millimetric precision.
- A photographic camera, preferably also attached to the mobile structure of the 3D scanner, although not necessarily. The main purpose of the photographic camera is to provide texture to the 3D image obtained through the scanner.
- A CPU (a Central Processing Unit) with a specific scanning software intended to obtain an improved 3D virtual model with respect to that obtained only with the scanner, since it makes it possible to easily add the texture of objects and faces by taking a photograph with the photographic camera and improve the image of the bust obtained from the scanner through easy steps that do not require expert handling.

The CPU further incorporates a specific software for capturing data and viewing and modifying the eyeglasses which, based on the data obtained from the scan, generates a graphical interface for adjusting the eyeglasses which allows the user to adjust the size to his/her taste and comfort. Said interface also makes it possible to measure the pantoscopic angle, the Galbe angle, the interpupillary distances and the distance to the optical centre.

Finally, the software is capable of generating, if desired, a file of the frame model chosen or designed in the graphical interface, in a compatible format for production by means of a 3D printer.

The CPU also has another specific software to obtain, based on any frame, a 3D image thereof, take the necessary measurements and generate a file compatible with the edgers available in the market, which will contain all the parameters needed to bevel any type of corrective lens, that is, to cut the glass so that it fits the shape of the frame.
- A screen connected to the CPU for viewing the 3D image and the graphical interface, which may be a tactile screen and/or be associated with a keyboard for interacting with the software.

Based on the above, the device operates as follows:
Whether it is secured to the floor or the ceiling, the support structure of the scanner and the photographic camera will be placed at the height of the standing patient/customer's bust. As soon as the structure is positioned and fixed at the correct height, photographs are taken with the photographic camera and the scanner will capture their data to produce the images of the customer/patient's bust through the mobile structure that is automatically or manually actuated.

The inner face of the mobile structure, as mentioned above, is designed to ensure outdoor light and possible reflections do not affect the capture of data by the scanner, as well as to ensure the lighting is appropriate, due to which it preferably incorporates lighting.

Once the mechanical process of taking the images is completed, the structure will return to its initial position.

An improved 3D image of the customer/patient is obtained with the data obtained from the scanner and the photographic camera and through the specific software described above. For its part, the second software programme developed makes it possible to obtain all the measurements for producing the specific glasses for each customer, to modify or design frames adapted to said customer and to serve as a tester with the actual image of existing frames.

Therefore, the main steps of the method for producing lenses and frames with personalised design and remote bevelling of corrective lenses proposed in the present invention comprises, at least, the following steps:
- obtaining morphological data from the patient/customer with millimetric measurements by means of a 3D scan of the bust;
- adding texture of objects and faces to photographs of the patient obtained with the photographic camera;
- generating a 3D virtual model of the frame in the photographic image;
- capturing data through the graphical interface by measuring the pantoscopic angle, the Galbe angle, the interpupillary distances and the distance to the optical centre for producing the lenses;
- generating a file compatible with an edger and containing all the parameters for bevelling any type of corrective lens adjusted to the shape of the frame.

In addition, when producing a new frame and after generating the 3D virtual model of the frame in the photographic image, it includes the steps of:
- viewing and modifying the frames, adjusting the size and design of the frames of the virtual model, and once chosen;
- generating the data file for the chosen frame in a compatible format for production by means of a 3D printer.

Furthermore, when the frame is an existing frame provided by the patient/customer for the bevelling of lenses adjusted to the frame, in the step of generating the 3D virtual model of the frame, the method comprises the steps of:
- obtaining a 3D image of the frame;
- capturing measurement data of said frame;
- and, subsequently, generating the file compatible with the edger, which contains all the parameters for bevelling any type of corrective lens adjusted to the shape of the frame.

It should be noted that, in addition, the optical device for morphological measurement makes it possible to perform the method described above by obtaining a 3D mesh of the patient/customer's bust, making the scanner rotate 360º as it moves vertically at the same time such that, in an alternative embodiment as a fixed structure to which the rest of the elements of the device are attached, it contemplates a profile secured to the wall equipped with a tensioner to give it stability and provide the necessary counterweight force to make the movements of the mobile structure as precise as possible, which is horizontally fixed at the required height as to be, by its distal end, above the patient/customer's head.

Furthermore, as a mobile structure, the device contemplates an L-shaped arm joined to said distal end of the profile that forms the fixed structure by means of a rotary part that makes it possible to rotate said arm 360° around the customer's bust.

Said rotary part is preferably formed by a group of gears that allow the mobile structure to rotate 360° as necessary for capturing the data from different perspectives. For this purpose, it is also preferably provided with a motor to automate this movement, in addition to the possibility of actuating the same manually or automatically. This rotary part is covered with a protective casing to make the device aesthetically pleasing.

On the other hand, the L-shaped arm constituting the mobile structure that rotates 360° around the patient/customer rotates from the shaft and gears with which it joins the aforementioned rotary part.

Furthermore, the L-shape of this arm is determined by a first segment which is approximately horizontal and parallel to the profile of the fixed structure, and a second segment which is approximately vertical and perpendicular to said structure. Said segments are connected to each other through an elbow joint, which is optionally mobile and forms an angle greater than 90°.

In any case, the vertical segment of the arm that makes up the mobile structure has a longitudinal guide, which covers most of its inner face and to which the scanner and the photographic camera are attached so that both have the possibility of moving along the length of the same.

This vertical movement has two purposes: the first is to adapt the scanner to the patient/customer's height, and the second is to provide 360° rotation with new perspectives from different heights or meridians, which makes it possible to obtain the 3D mesh described above with a special detail of the ears and nose.

The device with this combination of structures makes it possible to combine both movements, rotation on the horizontal plane and the vertical movement of the scanner along the guide of the L-shaped arm, which provides a wide range of data that will result in a more accurate design of the patient/customer's bust.

Both the rotation and vertical movement described above can be performed manually or through automatic actuating mechanisms that will be conveniently located in the rotary part joining the fixed structure and the L-shaped arm, as explained above, and in the guide of the vertical segment of the arm, which has a small mechanism formed by a motor and pulleys that allow the components in charge of capturing images to move vertically.

Optionally, the entire assembly described, and at least the rotary part and the L-shaped arm, can be surrounded and covered by a hollow cylinder, which surrounds the user's head in the form of a bell, and which inner face is adapted to ensure outdoor light and possible reflections do not affect the capture of data by the scanner, as well as to ensure the lighting is appropriate.

With this configuration, the optical device takes measurements with the scanner by rotating 360º around the patient/customer's bust at the same time as it moves vertically in different planes. This improvement makes it possible to obtain morphological data of the ears and the hollow they form with the head, which is very important to the extent it is the place which supports the temples of a frame and are therefore basic and essential for measuring the pantoscopic angle.

It should be noted that the anchorage point of the frame with the ears is critical for correctly establishing pupillary height and pantoscopic angle, which are essential measurements for personalising corrective lenses.

Thus, when taking the images by rotating 360º and moving in the vertical plane, it is possible to obtain data from the different horizontal meridians for producing a 3D mesh of the person's entire head, with special details for the nose and ears.

Furthermore, to ensure the image and virtual test of the scanned bust and the scanned frame are exactly the same as they would be in reality, and to ensure the virtual position of the eyeglasses on the nose matches the position achieved when the frame is actually placed on the nose, it is essential to have a highly precise 3D mesh. Taking measurements at different heights and meridians is essential for this purpose.

The method and optical device for morphological measurements described above for producing lenses and frames with personalised design and remote bevelling of corrective lenses therefore represents an innovation in structural and constituent characteristics unknown until now which, together with its practical value, make it worthy of the privilege of exclusivity applied for.

### DESCRIPTION OF THE DRAWINGS

To supplement the description being made, and in order to aid a better understanding of the characteristics of the invention, this specification is accompanied by a set of drawings representing the following in an illustrative rather than limiting manner:
Figure 1 represents a perspective view of a schematic representation of an example of the optical device object of the invention, which shows the main parts and elements comprising the same, as well as the configuration and arrangement thereof.
And figure 2 represents schematic perspective view of a representation of a second example of the optical device object of the invention, in a variant thereof with a different mobile structure, which shows the main parts and elements comprising the same, as well as the configuration and arrangement thereof.

### PREFERRED EMBODIMENT OF THE INVENTION

In view of the aforementioned figures and in accordance with the numerical references adopted, such figures represent a non-limiting example of the optical measurement device proposed herein, which comprises the parts and elements that are indicated and described in detail below.

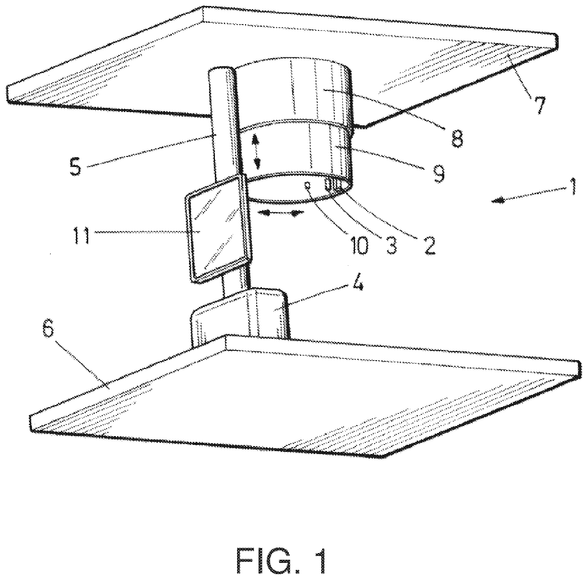

Thus, the aforementioned figure 1 shows how the device (1) in question comprises at least one 3D scanner (2) and one photographic camera (3) associated with a CPU (4) equipped with the specific software described in previous sections. These elements are preferably incorporated into a fixed structure (5) that provides the support and stability required for taking images of the patient/customer.

Specifically, the software incorporated by the CPU (4) consists of a first specific scanning software, which generates a 3D virtual model obtained from the scanner and perfected with the texture of objects and faces from photographs obtained with the photographic camera; and a second specific software for capturing data and viewing and modifying the frames, which, based on the data obtained from the scan, generates a graphical interface that makes it possible to measure the pantoscopic angle, the Galbe angle, the interpupillary distances and the distance to the optical centre for producing lenses, and also to adjust the size and design of the frames, and that, if desired, generates a file of the selected or designed frame in a format compatible for production with a 3D printer.

In the preferred embodiment, said fixed structure (5) constitutes an assembly in the form of a column mounted from the floor (6) up to the ceiling (7), although alternatively it could be mounted only on the floor or only from the ceiling, in any case contemplating at least one raised support (8) for placing the 3D scanner (2) and, preferably, also the photographic camera (3) at the height of the standing patient/customer's head.

Furthermore, in the preferred embodiment of the invention, the fixed structure (5) incorporates a mobile structure (9) attached under the raised support (8) to which the 3D scanner (2) and the photographic camera (3) are attached, which, equipped with automatic or manual actuating means, can at least move vertically to be placed at the required height, although, optionally it can also rotate on the horizontal plane around the customer's head or bust.

Preferably, this mobile structure (9) is a circular body that moves up and down and that rotates on itself around the patient/customer's head, and which inner face is adapted to ensure outdoor light and possible reflections do not affect the capture of data by the scanner, being optionally provided with lighting means (10).

The device (1) also comprises a screen (11) connected to the CPU (4), which may be a tactile screen and/or be associated with a keyboard, as an interface for controlling the operation of the different components and for interacting with the software. The CPU (4) is also connected to these components by means of the respective cabling, which is preferably hidden within the fixed structure (5).

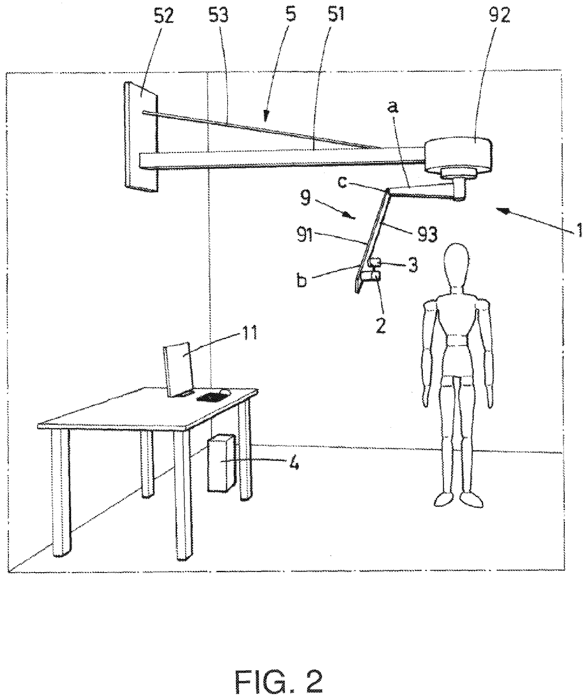

Furthermore, Figure 2 represents an alternative embodiment of the device (1) with a different configuration in the structure to which its components are attached. Specifically, in said variant the fixed structure (5) is a profile (51) secured to the wall (52), which is fixed horizontally at the required height as to be, by its distal end, above the patient/customer's head, and a mobile structure (9) consisting of an L-shaped arm (91) is joined to said distal end of the profile (51) by means of a rotary part (92) that makes it possible to rotate said arm (91) 360° around the attachment point therewith, and therefore around the patient/customer's bust, said arm (91) also having a longitudinal guide (93) to which the 3D scanner (2) and a photographic camera (3) are attached, such that both move vertically along the length thereof and such that the mobile structure (9) makes it possible to combine and simultaneously perform both movements, the 360° rotation on the horizontal plane and the vertical movement with the 3D scanner (2) and the photographic camera (3) around the patient/customer's bust.

Said rotary part is preferably formed by a group of gears allowing the arm (91) to rotate 360° and is optionally provided with a motor to automate this movement, in addition to the possibility of actuating the same manually or automatically. This rotary part (92) is preferably covered with a protective casing that hides said gears and drive motor.

For its part, the L-shaped arm (91) that performs the 360º rotation from the shaft and gears with which it joins the aforementioned rotary part (92) is formed by a first segment (a) which is approximately horizontal and parallel to the profile (51) of the fixed structure (5), and a second segment (b) which is approximately vertical and perpendicular to the profile (51), being joined to each other through an elbow joint (c), which is optionally mobile and forms an angle greater than 90°.

In any case, the second vertical segment (b) of the arm (91) has the longitudinal guide (93), to which both the 3D scanner (2) and the photographic camera (3) are attached, which consists of a longitudinal guide (3) covering most of its inner face.

Optionally, this guide (93) has a small mechanism formed by a motor and pulleys that enable the automated movement of the 3D scanner (2) and the photographic camera (3).

Finally, the profile (51) of the fixed structure (5) is equipped with a tensioner (53) to give it stability and provide the necessary counterweight force to allow the mobile structure (9) to move which, in turn, is covered by a hollow cylinder (not shown) covering at least the rotary part (92) and the L-shaped arm (91), which surrounds the user's head in the form of a bell, and which inner face is adapted to ensure outdoor light and possible reflections do not affect the capture of data by the scanner.

This description of the nature of the invention as well as its practical embodiments will allow any expert in the art to understand its scope and the advantages derived therefrom without further explanations. It is hereby provided that, by keeping its essential components, there can be other embodiments that may differ in detail from that indicated by way of example, and which can also be awarded the protection being sought as long as the fundamental principles of the invention are not altered, changed or modified.

## Claims

1. A METHOD FOR PRODUCING LENSES AND FRAMES, WITH PERSONALISED DESIGN AND REMOTE BEVELLING OF CORRECTIVE LENSES, **characterised in that** it comprises the following steps:
- obtaining morphological data from the patient/customer with millimetric measurements by means of a 3D scanning of the bust;
- adding texture of objects and face to photographs of the patient obtained with the photographic camera (3);
- generating a 3D virtual model of the frame in the photographic image;
- capturing data through a graphical interface measuring the pantoscopic angle, Galbe angle, interpupillary distances and the distance to the optical centre for producing the lenses;
- generating a file compatible with an edger and containing all the parameters for bevelling any type of corrective lens, adjusted to the shape of the frame.

2. THE METHOD FOR PRODUCING LENSES AND FRAMES, WITH PERSONALISED DESIGN AND REMOTE BEVELLING OF CORRECTIVE LENSES, according to claim 1, **characterised in that**, when the frame is new, after the step of generating the 3D virtual model of the frame, it comprises the steps of:
- viewing and modifying the frames, for adjusting the size and design thereof;
- generating a data file for the chosen frame in a compatible format for the production thereof by means of a 3D printer.

3. THE METHOD FOR PRODUCING LENSES AND FRAMES, WITH PERSONALISED DESIGN AND REMOTE BEVELLING OF CORRECTIVE LENSES, according to claim 1, **characterised in that**, when the frame is an existing frame, the step of generating the 3D virtual model of the frame comprises the steps of:
- obtaining a 3D image of the frame;
- capturing measurement data of said frame.

4. AN OPTICAL DEVICE FOR MORPHOLOGICAL MEASUREMENT for performing a method such as that described in claims 1 to 3 for producing lenses and frames, with personalised design and remote bevelling of corrective lenses, **characterised in that** it comprises at least one 3D scanner (2) and one photographic camera (3) associated with a CPU (4) connected to a tactile screen (11) and/or associated with a keyboard, as an interface for controlling operation, and a fixed structure (5), to which all the elements comprised in the device are incorporated, and which provides the support and stability required for taking images of the patient/customer with said 3D scanner (2) and said photographic camera (3).

5. THE OPTICAL DEVICE FOR MORPHOLOGICAL MEASUREMENT according to claim 4, **characterised in that** the fixed structure (5) constitutes an assembly in the form of a column mounted from the floor (6) up to the ceiling (7).

6. THE OPTICAL DEVICE FOR MORPHOLOGICAL MEASUREMENT according to claim 5, **characterised in that** the fixed structure (5) contemplates at least one raised support (8) for placing the 3D scanner (2) and the photographic camera (3), at the height of the standing patient/customer's head.

7. THE OPTICAL DEVICE FOR MORPHOLOGICAL MEASUREMENT according to claims 5 or 6, **characterised in that** the fixed structure (5) incorporates a mobile structure (9) to which the 3D scanner (2) and the photographic camera (3) are coupled, and which can at least move vertically to be positioned at the required height.

8. THE OPTICAL DEVICE FOR MORPHOLOGICAL MEASUREMENT according to claim 7, **characterised in that** the mobile structure (9) can also rotate on the horizontal plane around the customer's head or bust.

9. THE OPTICAL DEVICE FOR MORPHOLOGICAL MEASUREMENT according to claim 8, **characterised in that** the mobile structure (9) is a circular body that moves up and down and rotates on itself around the patient/customer's head.

10. THE OPTICAL DEVICE FOR MORPHOLOGICAL MEASUREMENT according to claim 9, **characterised in that** the inner face of the mobile structure (9) is an opaque and non-reflective surface designed to ensure outdoor light and possible reflections do not affect the capture of data by the scanner.

11. THE OPTICAL DEVICE FOR MORPHOLOGICAL MEASUREMENT according to claim 10, **characterised in that** inner face of the mobile structure (9) is provided with lighting means (10).

12. THE OPTICAL DEVICE FOR MORPHOLOGICAL MEASUREMENT according to claim 4, **characterised in that** the fixed structure (5) is a profile (51) with an anchor for securing the profile (51) to the wall (52), which is fixed horizontally at the required height as to stay, by its distal end, above the patient/customer's head, and a mobile structure (9) consisting of an L-shaped arm (91) is joined to said distal end by means of a rotary part (92) that makes it possible to rotate said arm (91) 360° around the attachment point therewith, and around the patient/customer's bust, said arm (91) also having a longitudinal guide (93) to which the 3D scanner (2) and a photographic camera (3) are attached, such that both move vertically along the length thereof and such that the mobile structure (9) makes it possible to combine and simultaneously perform both movements, the 360° rotation on the horizontal plane and the vertical movement with the 3D scanner (2) and the photographic camera (3) around the patient/customer's bust.

13. THE OPTICAL DEVICE FOR MORPHOLOGICAL MEASUREMENT according to claim 12, **characterised in that** the rotary part (92) is formed by a group of gears provided with a motor that automates the 360º rotation of the arm (91).

14. THE OPTICAL DEVICE FOR MORPHOLOGICAL MEASUREMENT according to claim 13, **characterised in that** the motor of the group of gears of the rotary part (92) is manually actuated.

15. THE OPTICAL DEVICE FOR MORPHOLOGICAL MEASUREMENT according to claim 13, **characterised in that** the motor of the group of gears of the rotary part (92) is automatically actuated.

16. THE OPTICAL DEVICE FOR MORPHOLOGICAL MEASUREMENT according to any of claims 12 to 15, **characterised in that** the L-shaped arm (91) is formed by a first segment (a) which is approximately horizontal and parallel to the profile (51) of the fixed structure (5), and a second segment (b) which is approximately vertical and perpendicular to the profile (51), being joined to each other through an elbow joint (c) that forms an angle greater than 90°.

17. THE OPTICAL DEVICE FOR MORPHOLOGICAL MEASUREMENT according to claim 16, **characterised in that** the elbow joint (c) joining the first and second segments of the L-shaped arm (91) is a mobile joint.

18. THE OPTICAL DEVICE FOR MORPHOLOGICAL MEASUREMENT according to claim 16 or 17, **characterised in that** the second vertical segment (b) of the arm (91) has the guide (93) to which the 3D scanner (2) and the photographic camera (3) are attached, which consists of a longitudinal guide (93) covering most of its inner face.

19. THE OPTICAL DEVICE FOR MORPHOLOGICAL MEASUREMENT according to any of claims 12 to 18, **characterised in that** the guide (93) of the arm (91) has a small mechanism formed by a motor and pulleys for the automated movement of the 3D scanner (2) and the photographic camera (3).

20. THE OPTICAL DEVICE FOR MORPHOLOGICAL MEASUREMENT according to any of claims 12 to 19, **characterised in that** the profile (51) of the fixed structure (5) is equipped with a tensioner (53).

21. THE OPTICAL DEVICE FOR MORPHOLOGICAL MEASUREMENT according to any of claims 12 to 20, **characterised in that** the mobile structure (9) is covered by a hollow cylinder covering at least the rotary part (92) and the L-shaped arm (91), which surrounds the user's head in the form of a bell, and which inner face is adapted to ensure outdoor light and possible reflections do not affect the capture of data by the scanner.
